# EUROPEAN PATENT SPECIFICATION

(11) **EP 0 956 336 B1**
(45) Date of publication and mention of the grant of the patent: **19.12.2007**
(21) Application number: 97943569.0
(22) Date of filing: 25.09.1997
(51) Int. Cl.: C12M 3/00, B24C 5/04

(54) **GAS-DRIVEN PARTICLE DELIVERY DEVICE**
GASBETRIEBENES GERÄT FÜR TEILCHENABGABE
DISPOSITIF DE DISTRIBUTION DE PARTICULES ENTRAINEES PAR UN GAZ

(30) Priority: 25.09.1996 US 719503
(43) Date of publication of application: 17.11.1999
(60) Divisional application: 07009145.9
(73) Proprietor: Powderject Vaccines, Inc., Madison, Wisconsin 53711 (US)
(72) Inventor: McCABE, Dennis, E., Middleton, WI 53562 (US); HEINZEN, Richard, J., North Freedom, WI 53951 (US)
(74) Representative: Price, Nigel John King
(86) International application number: PCT/US1997/017202
(87) International publication number: WO 1998/013470

(56) References cited:
- EP-A- 0 691 183
- WO-A-95/19799
- DE-A- 4 404 954
- US-A- 4 521 996

## Description

### Technical Field

The present invention relates to the field of delivering material into cells, more particularly to instruments for delivering material into cells using particle-mediated delivery techniques.

### Background of the Invention

Particle-mediated delivery of materials, particularly nucleic acid molecules, into living cells and tissue has emerged as an important tool of plant and animal biotechnology. Transient and long-term expression of genetic material delivered via particle-mediated techniques into target cells has been demonstrated in a wide variety of microorganisms, plants, and animals. Successful integration of DNA into germ cells has also been demonstrated using these techniques, and particle-mediated gene delivery instruments have been used to deliver other materials into cells, including pharmaceuticals and biopharmaceuticals such as proteins, peptides and hormones.

As the fundamentals of the technology of particle-mediated delivery have developed, attention has increasingly shifted toward the development of devices that offer the operator the ability to perform the particle-mediated gene delivery in a swift and convenient fashion. It is also desirable for the operation of the delivery device to be efficient and highly replicable.

One particular device, which uses compressed gas to accelerate carrier particles carrying biological materials into target tissue, is described in International Publication No. WO 95/19799.

The distribution or spread of carrier particles delivered from a particle-mediated delivery device, such as the device of WO 95/19799, can be critical in some applications, particularly when biological material is being delivered, for example genetic material. In applications where germline transformation events are desired, the need to control the delivery pattern of carrier particles is substantially more acute than in other applications, such as where only transient expression of introduced genetic material is needed. When an infrequent germline transformation event is desired, it is generally necessary to uniformly accelerate the particles toward a large target area to increase the likelihood that one or more target cells will be transformed.

Accordingly, even though the device of WO 95/19799 and other related instruments have been suited for their intended purposes, there remains a need to provide for heightened uniformity and distribution of particles delivered from such devices.

### Summary of the Invention

The invention is drawn to a gas-driven particle delivery device having elements which modify the flow of gas through the device, as defined in the appended claims. The device of the invention comprises means to isolate a charge of compressed gas in the instrument for each particle delivery operation. In one embodiment of the invention, a particle delivery device is provided which comprises a body having an acceleration passage formed therein. A rotational flow element is arranged within the acceleration passage and serves to impart a rotary motion in a flow of gas passing therethrough prior to, or after entry of that gas flow into an acceleration chamber which forms a downstream part of the acceleration passage.

In various aspects of the invention, the rotational flow element is used to impart a rotational motion on the gas flow prior to, during, and/or after the gas flow has contacted particles which are to be delivered from the device. The rotational flow element can be any feature or structure disposed within an acceleration passage, which feature or structure is capable of imparting rotational motion on a gas flow passing therethrough. One particular rotational flow element comprises a plug or baffle which is arranged within the acceleration passage at a position upstream from a source of particles. Another rotational flow element comprises a structure, such as a plurality of thin propeller-like vanes, arranged within the acceleration passage at a position downstream from a source of particles.

In another embodiment, a particle delivery device is provided which comprises a body with an elongate acceleration chamber formed therein. The device includes a mixing chamber that communicates with the inlet of the acceleration chamber, and an upstream gas chamber that communicates with the mixing chamber. A rotational flow element is arranged within the upstream gas chamber, and imparts a rotary motion in a flow of gas passing from the upstream gas chamber into the mixing chamber to form a vortex within the mixing chamber.

In yet a further embodiment, a particle delivery device is provided which comprises a body with an elongate acceleration chamber formed therein. The device includes a source of particles that is arranged adjacent to an inlet for the acceleration chamber. A turbulent flow element is arranged upstream from both the acceleration chamber and the source of particles, whereby such an element is used to create turbulence in a flow of gas passing therethrough prior to its contact with the source of particles. In one particular aspect of the invention, the turbulent flow element comprises a gas conduit arranged above the source of particles, wherein the gas conduit has a stepped portion of increased diameter.

In another embodiment of the invention, a particle delivery device is provided which comprises a flow constriction element that restricts flow of compressed gas into the device.

These and other objects, features and advantages of the present invention will become apparent from the following specification, read in light of the accompanying drawings.

### Brief Description of the Drawings

Figure 1 is an illustration depicting the general operation of a gas-driven particle delivery device.
Figures 2A-2C are schematic illustrations of the effect of variations in the geometry of the exit nozzle in the device of Figure 1.
Figure 3 is a pictorial representation of a particle delivery device embodying the present invention.
Figure 4 is a cut-away view of a portion of the instrument of Figure 3 showing a rotational flow element disposed within a portion of the device.
Figure 5 is a side plan view of a rotational flow element according to the present invention.
Figure 6 is a cut-away, exploded view of a portion of a particle delivery device comprising a rotational flow element.
Figure 7 is a non-exploded view of Figure 6.
Figure 8 is a plan view of the upstream face of the rotational flow element of the device of Figure 6.
Figure 9 is a side plan view of the rotational flow element of the device of Figure 6.
Figure 10 is a cut-away view of a turbulent flow element according to the present invention.
Figure 11 is a plan view of a flow constriction element according to the present invention.
Figure 12 is a graphical representation of the particle delivery study described in Example 1.

### Detailed Description of the Preferred Embodiment

Before describing the present invention in detail, it is to be understood that this invention is not limited to particular particle delivery devices or to particular carrier particles as such may, of: course, vary. It is also understood that different embodiments of the disclosed sample delivery modules and related devices may be tailored to the specific needs in the art. It is also to be understood that the terminology used herein is for the purpose of describing particular embodiments of the invention only, and is not intended to be limiting.

It must be noted that, as used in this specification and the appended claims, the singular forms "a", "an", and "the" include plural referents unless the content clearly dictates otherwise. Thus, for example, reference to "a particle" includes reference to mixtures of two or more particles, reference to "a therapeutic agent" encompasses one or more such agents, and the like.

### A. Definitions

Unless defined otherwise, all technical and scientific terms used herein have the same meaning as commonly understood by one of ordinary skill in the art to which the invention pertains. The following terms are intended to be defined as indicated below.

As used herein, the term "therapeutic agent" intends any compound or composition of matter which, when administered to an organism (human or animal) induces a desired pharmacologic, immunogenic, and/or physiologic effect by local, regional, and/or systemic action. The term therefore encompasses those compounds or chemicals traditionally regarded as drugs, vaccines, and biopharmaceuticals including molecules such as proteins, peptides, hormones, nucleic acids, gene constructs and the like.

More particularly, the term "therapeutic agent" includes compounds or compositions for use in all of the major therapeutic areas including, but not limited to, anti-infectives such as antibiotics and antiviral agents; analgesics and analgesic combinations; local and general anesthetics; anorexics; antiarthritics; antiasthmatic agents; anticonvulsants; antidepressants; antihistamines; anti-inflammatory agents; antinauseants; antineoplastics; antipruritics; antipsychotics; antipyretics; antispasmodics; cardiovascular preparations (including calcium channel blockers, beta-blockers, beta-agonists and antiarrythmics) ; antihypertensives; diuretics; vasodilators; central nervous system stimulants; cough and cold preparations; decongescants; diagnostics; hormones; bone growth stimulants and bone resorption inhibitors; immunosuppressives; muscle relaxants; psychostimulants; sedatives; tranquilizers; proteins, peptides and fragments thereof (whether naturally occurring, chemically synthesized or recombinantly produced); and nucleic acid molecules (polymeric forms of two or more nucleotides, either ribonucleotides (RNA) or deoxyribonucleotides (DNA) including both double- and single-stranded molecules, gene constructs, expression vectors, antisense molecules and the like).

Particles of a therapeutic agent, alone or in combination with other drugs or agents, are typically prepared as pharmaceutical compositions which can contain one or more added materials such as vehicles, and/or excipients. "Vehicles" and "excipients" generally refer to substantially inert materials which are nontoxic and do not interact with other components of the composition in a deleterious manner. These materials can be used to increase the amount of solids in particulate pharmaceutical compositions. Examples of suitable carriers include water, silicone, gelatin, waxes, and like materials. Examples of normally employed "excipients," include pharmaceutical grades of dextrose, sucrose, lactose, trehalose, mannitol, sorbitol, inositol, dextran, starch, cellulose, sodium or calcium phosphates, calcium sulfate, citric acid, tartaric acid, glycine, high molecular weight polyethylene glycols (PEG), and combinations thereof. In addition, it may be desirable to include a charged lipid and/or detergent in the pharmaceutical compositions. Such materials can be used as stabilizers, anti-oxidants, or used to reduce the possibility of local irritation at the site of administration. Suitable charged lipids include, without limitation, phosphatidylcholines (lecithin), and the like. Detergents will typically be a nonionic, anionic, cationic or amphoteric surfactant. Examples of suitable surfactants include, for example, Tergitol^{®} and Triton^{®} surfactants (Union Carbide Chemicals and Plastics, Danbury, CT), polyoxyethylenesorbitans, e.g., TWEEN^{®} surfactants (Atlas Chemical Industries, Wilmington, DE), polyoxyethylene ethers, e.g., Brij, pharmaceutically acceptable fatty acid esters, e.g., lauryl sulfate and salts thereof (SDS), and like materials.

When direct intracellular delivery is intended, therapeutic agents (or pharmaceutical preparations derived therefrom) can be coated onto carrier microparticles using a variety of techniques known in the art. Dense materials are preferred in order to provide microparticles that can be readily accelerated toward a target over a short distance, wherein the microparticles are still sufficiently small in size relative to the cells into which they are to be delivered. It has been found that microparticles having an average diameter of a few microns can readily enter living cells without unduly injuring such cells.

In particular, tungsten, gold, platinum and iridium microparticles can be used as carriers for therapeutic agents. Tungsten and gold are preferred. Tungsten microparticles are readily available in average sizes of 0.5 to 2.0 µm in diameter, and are thus suited for intracellular delivery. Although such microparticles have optimal density for use in particle delivery methods, and allow highly efficient coating with nucleic acids, tungsten may potentially be toxic to certain cell types. Thus, gold is a preferred material for use as carrier microparticles, as gold has a high density, is relatively inert to biological materials and resists oxidation, and is readily available in the form of spheres having an average diameter of from about C.2 to 3 µm. Spherical gold microparticles, or beads, in a size range of 1-3 microns have been successfully used in particle delivery technologies, as well as gold provided in the form of a microcrystalline powder having a measured size range of about 0.2 to 3 *µ*m.

### B. General Methods

In one embodiment, the invention is drawn to a component or topographical feature for use in a particle delivery device, which component or feature provides for an altered gas flow profile within the device. The altered gas flow, in turn, provides for a dramatic improvement in particle distribution in directions lateral to the major axis of the gas flow. In another embodiment, a component or topographical feature is used in a particle delivery device for providing a turbulent gas flow within the device. The turbulent flow allows for a more complete delivery of a payload of particles from the device. In yet a further embodiment, the invention is drawn to a means for limiting the amount of gas used to deliver particles from a gas-driven particle delivery device, wherein such limitation serves to significantly reduce the audible report associated with a particle delivery operation without a concomitant reduction in the efficacy of particle delivery from the device.

Various particle delivery devices suitable for delivering a particulate therapeutic agent, or microparticles coated with a therapeutic agent, are known in the art, and are all suitable for use in conjunction with the present invention. Such devices generally use a gaseous discharge to propel particles toward target cells. The particles can optionally be releasably attached to a movable carrier sheet, or removably attached to a surface along which a gas stream passes, lifting the particles from the surface and accelerating them toward the target. Examples of gaseous discharge devices are described in U.S. Patent No. 5,204,253 and in International Publication No. WO 95/19799.

Although the present invention is suitable for use with any particle delivery device, the invention is exemplified herein with reference to the device described in International Publication No. WO 95/19799. However, it is to be understood that any number of methods and devices similar or equivalent to those described herein can also be used in the practice of the present invention.

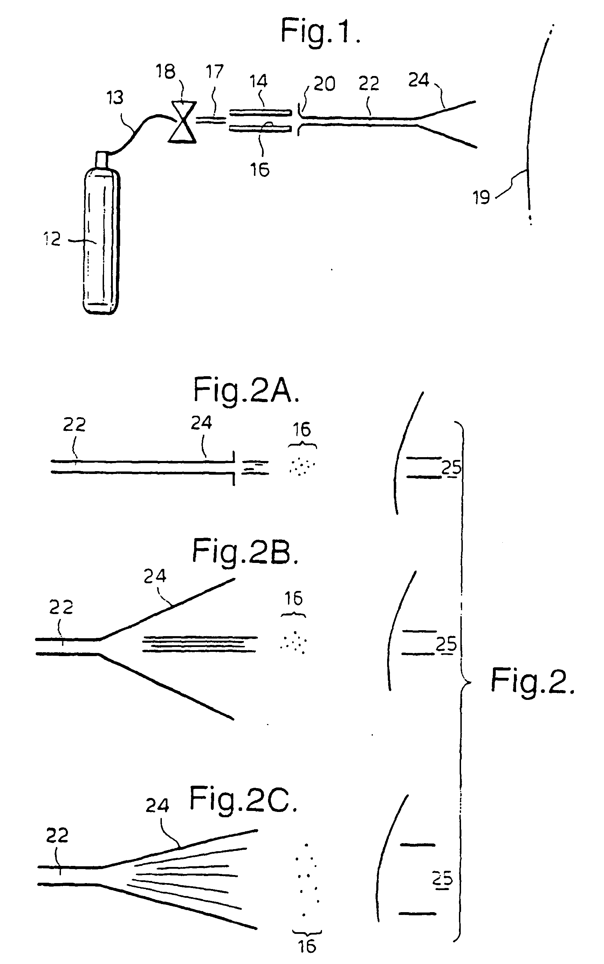

Turning now to the drawings, Figures 1 and 2 provide an illustration of the general method of operation of a particle delivery device such as that described in International Publication No. WO 95/19799. Components of the device are shown in slightly exploded view in some places for purposes of clarity. This particular depiction is intended to illustrate the basic operating principle of a particle delivery apparatus, rather than illustrate construction details.

Referring now to the device of Figure 1, a particle cartridge 14 is located within the particle delivery instrument. The particle cartridge 14 is an elongate concave or tubular structure that has a concave hollow passage passing through its center. A plurality of particles 16 are disposed on the interior of the cartridge. The particles, as discussed hereinabove, can be any particulate therapeutic agent or, preferably, can be comprised of small, dense carrier microparticles that are coated with a therapeutic agent, e.g., DNA or RNA, that is intended to be delivered into a target cell or tissue. Such microparticles may alternatively be coated with other types of biological materials such as peptides, cytokines, hormones, or proteins.

An actuator **18,** for example a gas valve, gate, or rupturable membrane, is located upstream of the particle cartridge and is in fluid communication with the interior of the cartridge **14** via an appropriate conduit **17.** The actuator is connected, by appropriate tubing generally indicated at **13,** with a source of compressed gas **12.** The source of compressed gas **12** can be a conventional commercial compressed gas tank, preferably containing an inert compressed gas such as helium. A reservoir of compressed gas is generally desirable between the gas source **12** and the actuator **18,** however, the tubing **13** can function as such a reservoir.

Adjacent to the particle cartridge **14** is an orifice **20** which provides fluid communication with the interior of an acceleration chamber **22** which communicates, in turn, with a conical exit nozzle **24.** The target, e.g., a patient, tissue surface, or cell, is designated **as 19** in the Figure.

In general operation, the actuator **18** is used to release a pulse of compressed gas through the device. A particle acceleration passage, disposed between the actuator **18** and the exit nozzle **24,** provides a path through which the released gas creates a gas stream traveling at significant speed. The gas stream accelerates through the particle acceleration passage and, as it passes through the interior of the particle cartridge **14,** dislodges the particles **16.** The accelerating gas stream, containing the dislodged particles, continues along the acceleration passage through the acceleration chamber **22,** and into the exit nozzle **24.**

One particularly important feature of the device of Figure 1 is the geometry of the exit nozzle **24.** Referring now to Figure 2, three different possible geometries of the exit nozzle **24** are illustrated schematically as Versions A, B, and C. Also depicted is the effect of these different exit nozzle geometries upon the delivery pattern of the particles **16.** In Version A, the exit nozzle **24** does not widen significantly toward the downstream end thereof. Thus, the exiting gas stream passes substantially linearly from the exi= nozzle **24,** and proceeds directly toward the target. As a result, the carrier particles continue in a relatively linear path and provide a focused delivery pattern that impacts a relatively narrow area **25** of the target. While the particles **16** diverge somewhat from their linear flight, the divergence is quite small and insignificant.

In Version B of Figure 2, the exit nozzle **24** has an exceedingly wide angle of conical taper toward the downstream terminus thereof. In this configuration, the gas stream exits the instrument fairly linearly, and the particles 16 do not disperse widely. Again, the particles impact a relatively compact portion **25** of the target.

A substantially different delivery pattern is obtained, however, when the angle of taper of the conical exit nozzle is less than a critical angle. This phenomenon is illustrated as Version C in Figure 2. In particular, as the accelerated gas stream passes into the exit nozzle, it creates, through a vortex action, a vacuum between the route of passage of the gas stream and the sides of the exit nozzle **24.** This vacuum causes the gas stream to be pulled outwardly in all directions perpendicular to the direction of travel of the gas stream. In this manner, the gas stream and the particles entrained within the gas stream are dispersed in a direction lateral to the major axis of the exit nozzle (i.e., the direction of travel of the particles). Thus, as can be seen in Version C of Figure 2, the gas stream passing out of the instrument is spread laterally over a wider area, thereby distributing the particles 16 over a wider area and providing an improved delivery profile over the surface area **25** of the target. This avoids overdosing any one small area of the target with the delivered particles.

The exact angle of taper of the conical exit nozzle **24** can be varied to accommodate use of different gas pressures and relative sizes for the acceleration chamber **22.** In an instrument which uses a commercial helium tank as the source of motive force, wherein the acceleration chamber **22** has a diameter of approximately 1.6 mm (1/16 inch) an exit nozzle which tapers from 1.6 mm (1/16 inch) to 1.7 cm (2/3 of an inch) over a span of 8.4 cm (3.3 inches) will provide a satisfactory particle distribution pattern which covers a target surface having a diameter of from about 1.6 mm (1/16 inch)to about 1.7 cm (2/3 of an inch). This represents over a 100-fold increase in the particle distribution pattern, with a concomitant 100-fold decrease in the particle distribution density.

In summary, then, the conical exit nozzle **24** of the device of WO 95/19799 can be configured significantly longer along its major axis than it is wide at either of its upstream or downstream termini in order to obtain a wider overall distribution of particles. In addition, by varying the pressure of the gas, the force with which particles impact the target **19** may be adjusted. At a minimum, however, the gas pressure provided by the source of motive force must be sufficient to dislodge the particles **16** from the cartridge **14.** At the same time, the gas pressure should not be so great as to damage the target **19.** When delivering coated carrier microparticles into intact animal skin using such devices, it has been found that a discharged gas stream will not harm the targeted skin surface. At higher gas pressures, some minor reddening of the skin may occur, generally at tolerable levels. A regulated gas pressure, such as that available from commercially available compressed helium tanks, has been found to be satisfactory for detaching the particles **16** from the cartridge **14,** and delivering the same into epidermal cells of a target animal without untoward damage to the target skin or cells. Lower pressures or higher pressures may work in particular applications, depending upon the density of the particles, the nature of the target surface, and the desired depth of particle penetration.

While use of the above-described exit nozzle geometries and operation parameters provides for a significant distribution of delivered particles over a target surface, the distribution pattern is not as uniform as is desired. In particular, even though the particle distribution provided by the device of WO 95/19799 is better than that achieved with any other compressed gas-driven device, the pattern is still characterized by a concentration of particles impacted in the center of the target area, with a laterally decreasing distribution of particles extending from that centralized area. Accordingly, it is a specific object of the invention to provide an element which serves to increase the uniformity of particle distribution obtainable from particle delivery devices.

In one particular embodiment of the invention, a rotational flow element is provided which can be positioned in a particle delivery device within an acceleration passage, for example at a location upstream from a source of particles (e.g., within the fluid conduit **17** of the device of WO 95/19799), or at a location downstream from a source of particles (e.g., within the acceleration chamber 22 of the device of WO 95/19799). The element can thus serve to re-channel all, or a portion, of the gas flow either prior to, or after its contacting the particles, thereby imparting a rotational motion on the gas flow prior to, during, and/or after the gas flow has contacted the particles. The element can be any feature or structure capable of imparting rotational motion on a gas flow. In one aspect of the invention, the element comprises one or more vanes, either disposed within, or depending from the interior surface of, a gas conduit within an acceleration passage. In one aspect of the invention, the vanes are positioned upstream of a source of particles to be delivered. In another aspect, the vanes are positioned downstream from a source of particles. The vane or vanes serve to re-channel at least a portion of the gas flow, forcing it to move or rotate about an axis. In yet another aspect, the element comprises a cylindrical plug or baffle disposed within a gas conduit that resides upstream from the source of particles. The plug or baffle contains one or more angled channels which allow an expanding flow of gas to pass therethrough so as to initiate a rotational flow in the expanding gas stream prior to, during, and/or after contact with the particles. These channels can be formed within the plug or baffle, disposed about the periphery of the plug or baffle such that a wall of the channel is provided by the gas conduit, or any combination of internal and peripheral channels can be employed. In any of such configurations, the rotational flow element serves to dramatically increase the lateral distribution of delivered particles, thus ensuring a more uniform particle distribution over the targeted area.

Not being bound by any particular theory, it is thought that imparting a rotation on the gas flow before, during, and/or after its contact with the particles assists in a turbulent intermixing of the particles within the gas, which in turn provides a better distribution of the particles within the expanding gas stream. Such rotational flow dynamics may also carry through to the exit nozzle of the particle delivery device, assisting in the formation of a laterally uniform dispersion of the delivered particles, possibly due to centrifugal forces. Regardless of the mechanism by which the result is achieved, the result is quite clear. The delivered particles are laterally dispersed from the major axis of the gas stream upon their exit from the particle delivery device, providing both a qualitatively and quantifiably measurable increase in the uniformity of particle distribution within a target area. In this manner, then, the present invention is broadly applicable for use in any gas-driven particle delivery device to provide enhanced uniformity in a particle distribution.

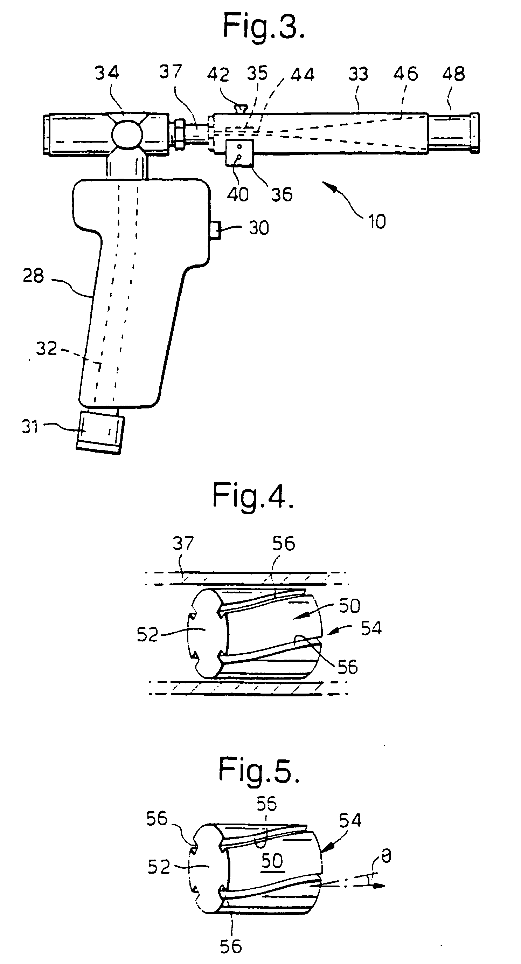

Referring to Figure 3, a particle delivery device similar to that of Figure 1 is generally indicated at **10.** The device **10** comprises a handle **28** through which an inlet conduit **32** passes. The inlet conduit **32** terminates at one end with a coupler **31** which allows connection of the device **10** with an associated source of compressed gas. A trigger **30,** situated on the handle **28,** allows for actuation of the device by releasing a flow of gas into the device from the associated source.

An upstream gas conduit **37** connects the handle **28** with an elongate body **33,** which body includes a cartridge chamber **35** capable of housing a particle cartridge. In the particular device of Figure 3, a cartridge holder **36,** mounted on the body **33,** houses several particle cartridges which are configured as cylindrical tubes coated on their interior surfaces with particles for delivery from the device. In operation, cartridges from the cartridge holder are individually brought into position within the cartridge chamber **35** such that they are disposed within the path of a gas flow passing through an acceleration passage that extends from the upstream gas conduit **37** through an acceleration chamber **44.** The acceleration chamber **44** terminates in an exit nozzle **46.** The rotational flow element of the present invention is preferably situated within the upstream gas conduit **37** such that it may impart a rotational motion on the gas stream passing therethrough prior to contact of that gas stream with the particles in the cartridge chamber.

Referring now to Figures 4 and 5, one particular rotational flow element is shown which comprises a baffle **50** having an upstream face **52** and a downstream face **54.** The baffle is configured as a plug which can be inserted within the upstream gas conduit **37** at a location adjacent =o the cartridge chamber **35.** The baffle **50** includes one or more gas channels **56** disposed in a radial array about its periphery. The baffle can be comprised of any suitably resilient material which is either machinable or molded, for example, metals, metal alloys and rigid polymeric materials. The gas channels extend along the length of the baffle in a direction that is substantially in the direction of gas flow through the gas conduit. However, as can be seen with particular reference to Figure 5, each channel can be canted or angled at a defined angle θ relative to the major axis of the baffle.

The particular angle θ can vary over a range of about 0-15°, and is preferably in the range of about 0-11°. A small angle θ of about 0-5° provides for deeper penetration of particles delivered from the particle delivery device. A medium angle θ of about 7-11° provides the broadest distribution of particles in a direction lateral to the direction of gas flow through the particle delivery device.

The baffle **50** can comprise external threads which cooperate with corresponding threading within the gas conduit **37,** or the baffle can have a substantially smooth external surface, for example where the baffle is compression fit within the gas conduit. In operation, a flow of gas released within the particle delivery device passes into the gas conduit **37** where it contacts the upstream face **52** of the baffle **50.** The expanding gas flow is then caused to pass through the gas channels **56,** which imparts a rotational motion on the gas flow proportional to the angle θ. This rotational gas flow then travels into a particle cartridge where it picks up the particles from the interior surface of the cartridge for delivery to a target surface.

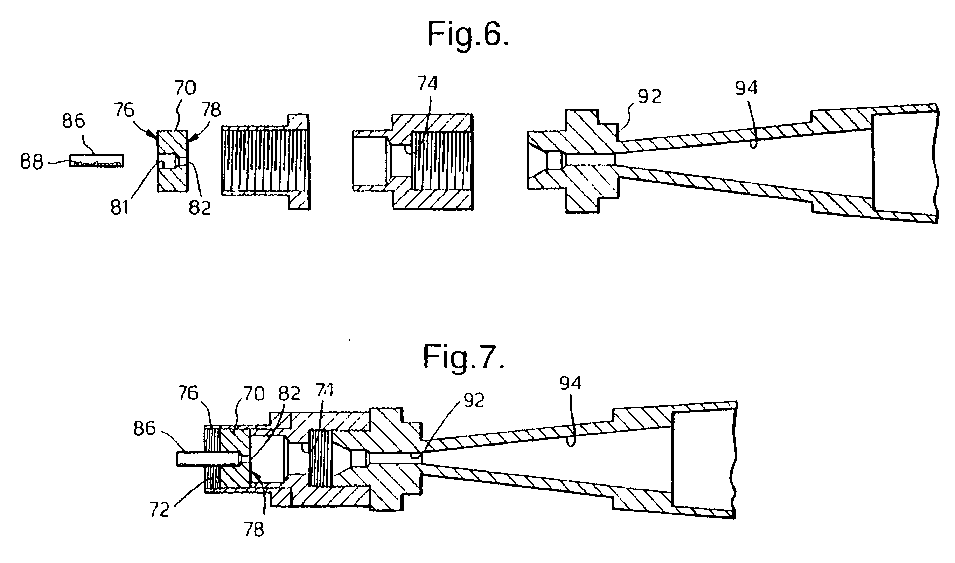

Referring now to Figures 6-9, a related rotational flow element baffle is generally indicated at **70.** The baffle **70** is arranged within a particle delivery device between an upstream chamber **72** which provides the initial chamber into which compressed gas is released, and a downstream mixing chamber **74.** The baffle **70** has an upstream face **76,** a downstream face **78,** and an outer surface **80.** A linear central bore **82** extends between the upstream and downstream faces **76** and **78,** wherein the central bore is coaxial with the major axis of the baffle **70.** An annular seat **84** in the upstream face **76** provides a recessed fitting which accepts and retains a cylindrical particle cartridge **86.** The annular seat **84** surrounds and is coaxially aligned with the central bore **82.**

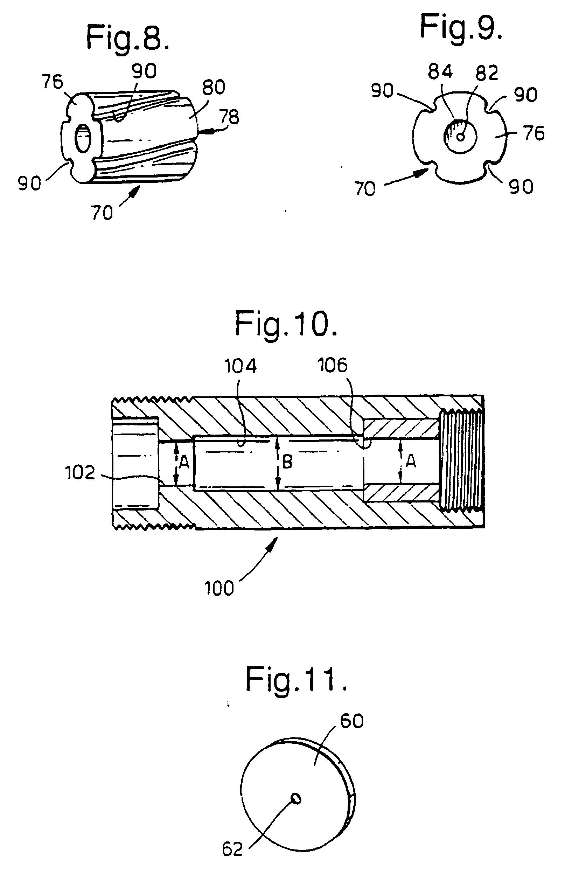

The particle cartridge **86** has a plurality of particles 88 coated on the interior surface thereof. As can be seen with reference to Figures 6 and 7, the particle cartridge **86** sits within the annular seat **84** and projects into the upstream chamber **72.** Referring now to Figures 8 and 9, the baffle **70** has one or more gas channels **90** in the outer surface **80** thereof, wherein the gas channels are disposed in a radial array about the central bore **82.** As described hereinabove, the gas channels can be canted or angled relative to the major axis of the baffle **70** in order to impart a rotational motion on gas flowing through the particle delivery device. The relative angle of the gas channels can range between 0-15° depending on the amount of rotational motion that is desired for the gas flow.

In operation, a flow of gas released into the upstream chamber **72** travels toward the upstream face **76** of the baffle **70,** with a portion of the gas flow entering the particle cartridge **86.** The reduced diameter of the central bore **82** relative to the diameter of the annular seat **84** and hence the particle cartridge **86,** restricts the amount of gas that can flow through the particle cartridge to a fixed percentage of the total gas flow. The bulk of the gas flow thus travels around the outer surface of the baffle **70** and through the gas channels **90.** This induces the formation of a vortex at a central point within the mixing chamber **74** where the gas flowing through the gas channels converges. An axial beam of particles **88** which have been dislodged from the interior surface of the particle cartridge **86** travels through the central bore **82** of the baffle in a substantially linear direction, and is thus delivered into the center of the vortex formed within the mixing chamber. As the vortexing gas flow and particle beam enter the acceleration chamber **92,** the spinning gas flow contacts the axial beam of particles which are then accelerated and centrifuged to impart a final trajectory as they pass through the nozzle **94,** distributing the particles uniformly over a target area.

Here again the baffle **70** can be comprised of any suitable machinable or moldable material that can withstand the force of a compressed gas flow suitable for delivery of particles through the particle delivery device. The baffle **70** can further include external threads on the outer surface **80** thereof in order to facilitate coupling to the upstream chamber **72.**

The rotational flow elements of the present invention serve to impart a rotational component in the flow of compressed gas stream passing through the particle delivery device. This rotational flow component causes some surprising results in the operation of particle delivery devices. One unexpected result is that a device fitted with a rotational flow element, referred to here sometimes as a "spinner" instrument, is more effective in picking up particles from the interior of the particle cartridge **14**. However, the rotational flow elements of the present invention provide their greatest benefit by substantially enhancing uniformity in particle distribution over a target area.

In another embodiment of the invention, a turbulent flow element is provided which can be positioned in a particle delivery device at a location upstream from a source of particles (e.g., within the fluid conduit **17** of the device of WO 95/19799). The element serves to perturb all, or a portion, of the gas flow prior to its contacting the particles, thereby improving the reliability of particle release from the particle source. The turbulent flow element can thus be any feature or structure capable of disrupting a gas flow in such a way as to impart a flow turbulence. In one aspect of the invention, the element comprises one or more topographical features disposed on the interior surface of an upstream gas chamber. Such features can comprise a ridge, shoulder, groove, corrugation, or any combination thereof, which features serve to disrupt or perturb the flow of gas passing through a chamber or conduit. In a particular aspect of the invention, the turbulent flow element comprises a small step in the interior surface of a cylindrical gas chamber which establishes a chamber area of slightly increased diameter bounded by areas of lesser diameters, through which areas an expanding gas flow passes prior to contacting a source of particles.

Referring now to Figure 10, a turbulent flow element is generally indicated at **100.** The flow element **100** is adapted for insertion into a particle delivery device, wherein it accepts a released gas flow through an upstream opening **102** which has a first diameter **A.** As the gas flow proceeds through the element, it enters a stepped portion **104** having a slightly greater diameter **B.** The stepped portion of the element **100** extends along a substantial portion of the overall length of the element and is bounded on its downstream side with a downstream opening **106** having a diameter **A** equal to that of the upstream opening. The stepped portion **104** of the element is sufficient to introduce a turbulence in the gas flow prior to its contact with a source of particles arranged adjacent to the downstream opening **106.** This turbulence improves the reliability of particle release from the particle source, improving particle delivery efficiency. In a particular embodiment, the diameter A of the upstream and downstream openings **102** and **106** is about 6.4 mm (0.250 inches), and the diameter B of the stepped portion **104** is about 7.1 mm (0.280 inches), providing a step of about 0.7 mm (0.03 inches).

In yet a further embodiment of the invention, a flow constriction element is provided for use in a particle delivery device. Referring to Figure 3, the constriction element is configured for insertion into the particle delivery device adjacent to the point of coupling between the device and the associated source of compressed gas. In particular, the constriction element can be inserted at the junction between the inlet tube **32** and the connector **31.** The constriction element is generally comprised of a disk of flexible or resilient material sized to restrict passage of gas into the delivery device to a small orifice. The orifice can be quite small, for example, an orifice of about 200 to 250 µm has been found to be sufficient. Referring now to Figure 11, a flow constriction element constructed according to the present invention is generally indicated at **60.** The element is configured as a disk having an orifice **62** passing therethrough.

The purpose of the restriction provided by the orifice **62** is to isolate an aliquot of compressed gas in the instrument for each particle delivery operation. With particular reference to the device described in International Publication No. WO 95/19799, the source of compressed gas supplies additional compressed gas through the instrument at all times, not just during delivery operations. As it turns out, more gas is delivered into the instrument upon actuation than is actually required for effective delivery of the particles to a target surface. In fact, the excess gas travelling through the instrument merely adds to the impact of the delivery operation on the target without providing a corresponding benefit. By providing the flow constriction element **60** in the device, the compressed gas bleeds through the orifice into the interior of the gene delivery instrument between delivery operations. When the device is actuated, then, there is a charge or aliquot of compressed gas already present in the instrument itself, occupying the various chambers and conduits within the device. This charge of compressed gas is, as it turns out, sufficient to deliver the particles from the particle source to the target surface. When the charge of compressed gas has been thus released, the flow restriction provided by the flow constriction element 60 prevents an additional volume of compressed gas from flowing through the instrument. After the delivery operation, the compressed gas will again recharge the instrument until a pressure equilibrium is reached.

In other related aspects of the invention, the concept of providing a single aliquot (charge) of gas within the particle delivery device to discharge a payload of particles can be carried out using other mechanisms. For example, instead of using a flow constriction element such as the element **60**, a combination of valves can be used to achieve the same effect. In one particular arrangement, an inlet valve can be provided which is closed during operation of the instrument, and then opened when the instrument is not being operated in order to charge the instrument for the subsequent operation. For example, if an electric or solenoid operated valve is used as the main valve for the instrument, two valves can be operated alternately wherein an inlet valve is closed whenever the main valve is opened, and the inlet valve is opened whenever the main valve is closed.

The benefits of the flow constriction element are several fold. For example, the audible report created by operation of the instrument is dramatically reduced. At 3.45 MPa (500 psi) of compressed gas, a typical particle delivery device will generate an approximately 103 dB report upon discharge, whereas the same instrument having the present flow constriction element generates only an 88 dB report upon discharge. There is also a perceptible dampening of the sensory feel of the gas discharge from a particle delivery device when the present constriction element is employed. Furthermore, there is less damage to sensitive target tissues or cells when the flow constriction element is used.

It is specifically intended herein that the various embodiments of the invention can be used alone or in any combination. In this regard, each embodiment is capable of independently providing a unique and advantageous improvement in the performance of a particle delivery device. However it is particularly advantageous to use both a rotational flow element and a flow constriction element to obtain optimum results.

### C. Experimental

Below are examples of specific embodiments for carrying out the present invention. The examples are offered for illustrative purposes only, and are not intended to limit the scope of the present invention in any way.

Efforts have been made to ensure accuracy with respect to numbers used (*e.g.,* amounts, temperatures, etc.), but some experimental error and deviation should, of course, be allowed for.

### Example 1

### Particle Delivery Distribution

In order to assess the effect of a rotational flow element on the performance of a particle delivery device, the following study was carried out. A particle delivery device, such as the "gene gun" described in International Publication No. WO 95/19799, was used to deliver gold microparticles into a Parafilm^{®} block covered by a Mylar^{®} film. This arrangement approximates a typical skin tissue target surface, and is sufficiently opaque to allow visual and/or optical assessments of the particle distribution in a target area. In the present study, a Model GS300 optical reader (Hoefer Scientific) was used to generate a graphical representation of the distribution of the gold microparticles in a transect across the center of the particle spread.

Six different particle deliveries were conducted, using identical particle payloads which were delivered from an ACCELL^{®} particle delivery device (PowderJect Vaccines, Madison, WI) operated at 400 psi helium. Each delivery was conducted using a rotational flow element that provided a different degree of spin (0°, 2°, 3.5°, 5°, 7°, and 11°).

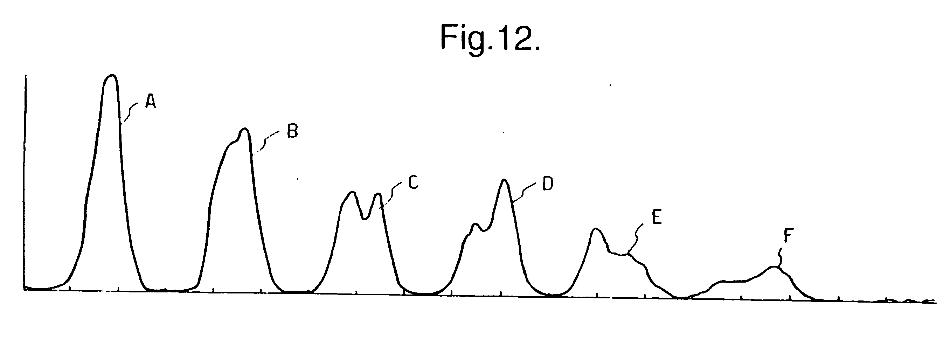

The results of the study are presented in the graph of Figure 12. In the graph, the ordinate value represents particle density, while the abscissa value represents particle spread. As can be seen in Figure 12, the curve **A** (obtained from the particle delivery using a rotational flow element that provides 0° spin) is both tall and narrow, demonstrating that the particles did not spread laterally and were concentrated in the center of the target area. In contrast, the curve C (obtained from the particle delivery using a rotational flow element that provides 3.5° spin) is much wider relative to curve **A**, indicating a higher particle spread over the target area. In addition, curve C is lower, indicating that there is not a particularly high concentration of particles in the center of the target area. The curve F (obtained from the particle delivery using a rotational flow element that provides 11° spin) shows that this higher degree of rotation significantly disperses the particles over the entire target area.

Subsequent gene delivery experiments into the skin of animals confirms that the rotational flow element improves operation of particle delivery devices. The use of the element was shown to provide comparable levels of reporter gene expression in test animals when compared with parallel experiments using devices without the flow elements. In addition, it was observed that the occurrence of erythremia in the skin of test animals was significantly reduced when using the rotational flow element in the particle delivery devices.

Accordingly, novel gas flow modifying elements for use with particle delivery devices have been described. Although preferred-embodiments of the subject invention have been described in some detail, it is understood that obvious variations can be made without departing from the scope of the invention as defined by the appended claims.

## Claims

1. A device (10) adapted to deliver particles into a target cell or tissue, said device comprising:
a body (33) having an elongate acceleration chamber formed therein, said chamber having an inlet and an outlet;
a source of compressed gas (12) coupled to the body for delivery of a gas flow into the inlet of the acceleration chamber; and
means to isolate a charge of compressed gas in the instrument for each particle delivery operation, such that additional volume of compressed gas from said source of compressed gas (12) is prevented from flowing through the instrument during particle delivery.

2. The device of claim 1, wherein said means to isolate a charge of compressed gas allows gas from said source (12) to recharge the instrument after the delivery operation.

3. The device of claim 1 or 2, wherein said means to isolate a charge of compressed gas comprises a flow constriction element (60) that limits flow of gas from the source (12) into the body.

4. The device of claim 3, wherein the flow constriction element (60) comprises a plug having an orifice (62) passing therethrough.

5. The device of claim 1 or 2, wherein said means to isolate a charge of compressed gas comprises a combination of valves.

6. The device of claim 5, wherein said valves comprise an inlet valve arranged to be closed during the operation of the instrument, and then opened when the instrument is not being operated in order to charge the instrument for the subsequent operation.

7. The device of any one of claims 1 to 6, further comprising a rotational flow element (50) arranged upstream from the inlet of the acceleration chamber, wherein said rotational flow element imparts a rotary motion in a flow of gas passing therethrough prior to entry of said gas flow into the acceleration chamber.

## Patentansprüche

1. Vorrichtung (10), die zum Befördern von Partikeln in eine Zielzelle oder ein Zielgewebe ausgelegt ist, wobei die Vorrichtung umfasst:
- ein Gehäuse (33) mit einer darin ausgebildeten länglichen Beschleunigungskammer, wobei die Kammer einen Einlass und einen Auslass aufweist;
- eine mit dem Gehäuse verbundene Druckgasquelle (12) zum Befördern eines Gasstroms in den Einlass der Beschleunigungskammer; und
- Mittel zum Isolieren einer Druckgasfüllung in dem Instrument für jeden Partikelbeförderungsvorgang, so dass während der Partikelbeförderung ein Strömen zusätzlichen Druckgasvolumens aus der Druckgasquelle (12) durch das Instrument verhindert wird.

2. Vorrichtung nach Anspruch 1, **dadurch gekennzeichnet, dass** das Mittel zum Isolieren einer Druckgasfüllung Gas aus der Quelle (12) das Instrument nach dem Beförderungsvorgang nachfüllen lässt.

3. Vorrichtung nach Anspruch 1 oder 2, **dadurch gekennzeichnet, dass** das Mittel zum Isolieren einer Druckgasfüllung ein Element zur Strömungsdrosselung (60) umfasst, welches das Strömen von Gas von der Quelle (12) in das Gehäuse beschränkt.

4. Vorrichtung nach Anspruch 3, **dadurch gekennzeichnet, dass** das Element zur Strömungsdrosselung (60) einen Stopfen mit einer durch diesen verlaufenden Auslassöffnung (62) umfasst.

5. Vorrichtung nach Anspruch 1 oder 2, **dadurch gekennzeichnet, dass** das Mittel zum Isolieren einer Druckgasfüllung eine Kombination von Ventilen umfasst.

6. Vorrichtung nach Anspruch 5, **dadurch gekennzeichnet, dass** die Ventile ein dafür ausgelegtes Einlassventil umfassen, dass es während der Betätigung des Instruments geschlossen ist und dann geöffnet wird, wenn das Instrument nicht betätigt wird, um das Instrument für die folgende Betätigung zu füllen.

7. Vorrichtung nach einem der Ansprüche 1 bis 6, welche weiterhin ein stromaufwärts des Einlasses der Beschleunigungskammer angeordnetes Wirbelströmungselement (50) umfasst, **dadurch gekennzeichnet, dass** das Wirbelströmungselement eine Drehbewegung in einem durch dieses strömenden Gasstrom vor dem Eindringen des Gasstroms in die Beschleunigungskammer ausübt.

## Revendications

1. Dispositif (10) adapté pour délivrer des particules dans une cellule ou un tissu cible, ledit dispositif comprenant:
un corps (33) comportant une chambre d'accélération allongée formée à l'intérieur de celui-ci, ladite chambre comportant un orifice d'entrée et un orifice de sortie ;
une source de gaz comprimé (12) couplée au corps pour la délivrance d'un écoulement de gaz dans l'orifice d'entrée de la chambre d'accélération ; et
des moyens pour isoler une charge de gaz comprimé dans l'instrument pour chaque opération de délivrance de particules, de telle sorte qu'un volume additionnel de gaz comprimé venant de ladite source de gaz comprimé (12) soit empêché de s'écouler à travers l'instrument durant la délivrance de particules.

2. Dispositif selon la revendication 1, dans lequel lesdits moyens pour isoler une charge de gaz comprimé permettent à un gaz venant de ladite source (12) de recharger l'instrument après l'opération de délivrance.

3. Dispositif selon la revendication 1 ou 2, dans lequel lesdits moyens pour isoler une charge de gaz comprimé comprennent un élément d'étranglement d'écoulement (60) qui limite l'écoulement de gaz à partir de la source (12) dans le corps.

4. Dispositif selon la revendication 3, dans lequel l'élément d'étranglement d'écoulement (60) comprend un bouchon comportant un orifice (62) traversant celui-ci.

5. Dispositif selon la revendication 1 ou 2, dans lequel lesdits moyens pour isoler une charge de gaz comprimé comprennent une combinaison de soupapes.

6. Dispositif selon la revendication 5, dans lequel lesdites soupapes comprennent une soupape d'entrée agencée de façon à être fermée durant le fonctionnement de l'instrument, puis ouverte lorsque l'instrument n'est pas en fonctionnement afin de charger l'instrument pour l'opération suivante.

7. Dispositif selon l'une quelconque des revendications 1 à 6, comprenant de plus un élément d'écoulement par rotation (50) disposé en amont de l'orifice d'entrée de la chambre d'accélération, ledit élément d'écoulement par rotation communiquant un mouvement de rotation dans un écoulement de gaz traversant celui-ci avant l'entrée dudit écoulement de gaz dans la chambre d'accélération.
